# EUROPEAN PATENT APPLICATION

(11) **EP 4 679 366 A1**
(43) Date of publication of application: **14.01.2026**
(21) Application number: 25185495.6
(22) Date of filing: 26.06.2025
(51) Int. Cl.: G06T 7/246, G06T 7/33, G06T 7/55, G06T 11/00

(54) **MEDICAL IMAGE PROCESSING METHOD AND MEDICAL IMAGING SYSTEM**

(30) Priority: 09.07.2024 CN 202410918166
(71) Applicant: GE Precision Healthcare LLC, Waukesha, WI 53188 (US)
(72) Inventor: QI, Buer, Milwaukee, 53226 (US); WANG, Dejun, Milwaukee, 53226 (US); ZHOU, Aizhen, Milwaukee, 53226 (US); BERNARD, Sylvain, Milwaukee, 53226 (US)
(74) Representative: Kilburn & Strode LLP

(57) **Abstract**

Embodiments of the present application provide a medical image processing method and a medical imaging system. The method comprises: acquiring a plurality of pieces of image data of a subject under examination; performing image registration on the plurality of pieces of image data; and performing at least one of the following processes according to the image registration result: performing image reconstruction, and generating indication information indicating the motion of the subject under examination.

## Description

### TECHNICAL FIELD

Embodiments of the present application relate to the technical field of medical imaging, and in particular relate to a medical image processing method and a medical imaging system.

### BACKGROUND

Tomosynthesis (TOMO) is an important medical imaging technique. In this technique, an X-ray source moves along a curved or linear path relative to a subject under examination so as to acquire a plurality of 2D projection images of a body part from a plurality of angles during scanning, and a 3D slice image can be acquired by reconstructing the plurality of 2D projection images, thereby facilitating improvement in diagnosis efficiency and accuracy. TOMO is commonly used in mammography and radiography (DR) systems.

### SUMMARY

Embodiments of the present application provide a medical image processing method and a medical imaging system.

According to an aspect of the embodiments of the present application, a medical image processing method is provided, comprising:

Acquiring a plurality of pieces of image data of a subject under examination;

Performing image registration on the plurality of pieces of image data; and

Performing at least one of the following processes according to the image registration result: performing image reconstruction, and generating indication information indicating the motion of the subject under examination.

According to an aspect of the embodiments of the present application, a medical imaging system is provided, comprising:

An acquisition device, acquiring a plurality of pieces of image data of a subject under examination; and

A controller, configured to execute the medical image processing method of said aspect.

According to an aspect of the embodiments of the present application, a computer-readable storage medium is provided, the computer-readable storage medium comprising instructions, and the instructions, when executed, causing a processor to be configured to execute the medical image processing method of said aspect.

With reference to the following description and drawings, specific embodiments of the examples of the present application are disclosed in detail, and the means by which the principles of the examples of the present application can be employed are illustrated. It should be understood that the embodiments of the present application are therefore not limited in scope. Within the scope of the spirit and clauses of the appended claims, the embodiments of the present application include many changes, modifications, and equivalents.

### BRIEF DESCRIPTION OF THE DRAWINGS

The included drawings are used to provide further understanding of the examples of the present application, which constitute a part of the description and are used to illustrate the embodiments of the present application and explain the principles of the present application together with textual description. Evidently, the drawings in the following description are merely some examples of the present application, and a person of ordinary skill in the art may obtain other embodiments according to the drawings without involving inventive skill. In the drawings:
FIG. 1 is a schematic diagram of a medical imaging system of an embodiment of the present application;
FIG. 2 is a schematic diagram of a tomosynthesis mode according to an embodiment of the present application;
FIG. 3 is a schematic diagram of a reconstructed image affected by motion of a subject according to an embodiment of the present application;
FIG. 4 is a schematic diagram of a reconstructed image affected by motion of a subject according to an embodiment of the present application;
FIG. 5 is a schematic diagram of an existing reconstructed image;
FIG. 6 is a schematic diagram of a medical image processing method according to an embodiment of the present application;
FIG. 7 is a schematic diagram of image registration according to an embodiment of the present application;
FIG. 8 is a schematic diagram of image registration according to an embodiment of the present application;
FIG. 9 is a schematic diagram of step 602 according to an embodiment of the present application;
FIG. 10 is a schematic diagram of an image registration method according to an embodiment of the present application;
FIG. 11 is a schematic diagram of indication information according to an embodiment of the present application;
FIG. 12 is a schematic diagram of indication information according to an embodiment of the present application;
FIG. 13 is a schematic diagram of a medical image processing method according to an embodiment of the present application;
FIG. 14 is a schematic diagram of a graphical user interface according to an embodiment of the present application;
FIG. 15 is a schematic diagram of a reconstructed image according to an embodiment of the present application; and
FIG. 16 is a schematic diagram of a medical image processing apparatus according to an embodiment of the present application.

### DETAILED DESCRIPTION

The foregoing and other features of the examples of the present application will become apparent from the following description and with reference to the drawings. In the description and drawings, specific embodiments of the present application are disclosed in detail, and part of the embodiments in which the principles of the examples of the present application may be employed are indicated. It should be understood that the present application is not limited to the described embodiments. On the contrary, the examples of the present application include all modifications, variations, and equivalents which fall within the scope of the appended claims.

In the examples of the present application, the terms "first" and "second" and so on are used to distinguish different elements from one another by their title, but do not represent the spatial arrangement, temporal order, or the like of the elements, and the elements should not be limited by said terms. The term "and/or" includes any one of and all combinations of one or more associated listed terms. The terms "comprise", "include", "have", etc., refer to the presence of stated features, elements, components, or assemblies, but do not exclude the presence or addition of one or more other features, elements, components, or assemblies. The terms "connect", "connected", "couple", as well as other similar terms to which the embodiments of the present application relate are not limited to physical or mechanical connections, but may include electrical connections, whether directly connected or indirectly connected.

In the examples of the present application, the singular forms "a" and "the" or the like include plural forms, and should be broadly construed as "a type of" or "a kind of" rather than being limited to the meaning of "one". Furthermore, the term "the" should be construed as including both the singular and plural forms, unless otherwise specified in the context. In addition, the term "according to" should be construed as "at least in part according to...", and the term "based on" should be construed as "at least in part based on...", unless otherwise clearly specified in the context.

The features described and/or illustrated for one embodiment may be used in one or more other embodiments in an identical or similar manner, combined with features in other embodiments, or replace features in other embodiments. The term "include/comprise" when used herein refers to the presence of features, integrated components, steps, or assemblies, but does not preclude the presence or addition of one or more other features, integrated components, steps, or assemblies.

Medical imaging systems described herein include, but are not limited to, tomosynthesis imaging systems, computed tomography (CT) systems, positron emission tomography (PET) systems, single-photon emission computed tomography (SPECT) systems, ultrasound imaging systems, and magnetic resonance imaging (MRI). Thus, the present disclosure may be used in any time-series medical imaging system to identify any abnormal change between images attributable to patient motion during a scan.

In the embodiments of the present application, the term "subject under examination" may include any object being imaged. The term "projection data" is interchangeable with "projection image", "sinogram", and "projection image data".

The following description is provided using an example in which the medical imaging system is based on a suspended X-ray imaging system, but the embodiments of the present application are not limited thereto.

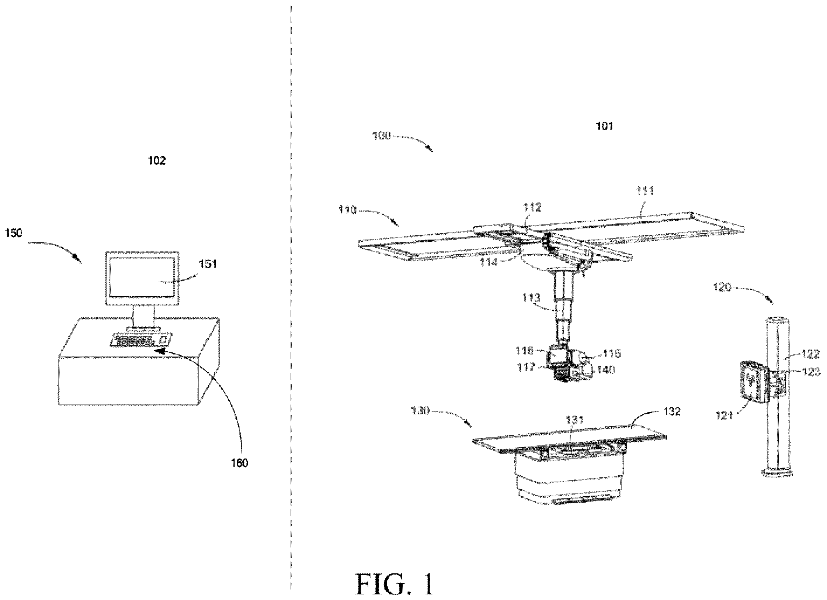

FIG. 1 is a medical imaging system 100 of an embodiment of the present application. As shown in FIG. 1, the medical imaging system 100 includes a suspension apparatus 110, a wall stand apparatus 120 and an examination bed apparatus 130 provided in a scanning room 101, and a control apparatus 150 provided in a control room 102. The suspension apparatus 110 includes a longitudinal guide rail 111, a transverse guide rail 112, a telescopic cylinder 113, a sliding member 114 and a tube assembly 115.

For ease of description, in the present application, an x-axis, y-axis, and z-axis are defined as the x-axis and the y-axis being located in a horizontal plane and being perpendicular to one another, and the z-axis being perpendicular to the horizontal plane. Specifically, the direction in which the longitudinal guide rail 111 is located is defined as the x-axis, the direction in which the transverse guide rail 112 is located is defined as the y-axis direction, and the direction of extension of the telescopic cylinder 113 is defined as the z-axis direction, and the z-axis direction is the vertical direction.

The longitudinal guide rail 111 and the transverse guide rail 112 are perpendicularly arranged, wherein the longitudinal guide rail 111 is mounted on a ceiling, and the transverse guide rail 112 is mounted on the longitudinal guide rail 111. The telescopic cylinder 113 is used to carry the tube assembly 115.

The sliding member 114 is disposed between the transverse guide rail 112 and the telescopic cylinder 113. The sliding member 114 may include components such as a rotary shaft, a motor, and a reel. A motor can drive the reel to rotate around the rotary shaft, which in turn drives the telescopic cylinder 113 to move along the z axis and/or slide relative to the transverse guide rail. The sliding member 114 can slide relative to the transverse guide rail 112, that is, the sliding member 114 can drive the telescopic cylinder 113 and/or the tube assembly 115 to move in the y-axis direction. Furthermore, the transverse guide rail 112 can slide relative to the longitudinal guide rail 111, which in turn drives the telescopic cylinder 113 and/or the tube assembly 115 to move in the x-axis direction.

The telescopic cylinder 113 includes a plurality of columns having different inner diameters, and the plurality of columns may be sleeved sequentially from bottom to top in columns located thereon to thereby achieve telescoping. The telescopic cylinder 113 can be telescopic (or movable) in the vertical direction, that is, the telescopic cylinder 113 can drive the tube assembly to move along the z-axis direction. The lower end of the telescopic cylinder 113 is further provided with a rotating part, and the rotating part may drive the tube assembly 115 to rotate.

The tube assembly 115 includes an X-ray tube, and the X-ray tube may produce X-rays and project the X-rays to a patient's intended region of interest (ROI). Specifically, the X-ray tube may be positioned adjacent to a beam limiter, and the beam limiter is used to align the X-rays with the patient's intended region of interest. At least part of the X-rays may be attenuated by means of the patient and may be incident on a detector 121/131. In addition, not shown in the drawings, the X-ray imaging system may also include a positionally flexible handheld detector for imaging some joints or infants.

The suspension apparatus 110 further includes a beam limiter 117, which is usually mounted below the X-ray tube, and the X-rays emitted by the X-ray tube irradiate on the body of a subject under examination by means of an opening of the beam limiter 117. The size of the opening of the beam limiter 117 determines an irradiation range of the X-rays, namely, the size of a region of an exposure field of view (FOV). The position of the X-ray tube and beam limiter 117 in the transverse direction determines the position of the exposure FOV on the body of the subject under examination. It is well known that X-rays are harmful to the human body, so it is necessary to control the X-rays so that the X-rays only irradiate the site of the subject under examination that needs to be examined, namely, the region of interest (ROI).

The suspension apparatus 110 further includes a tube control apparatus (console) 116. The tube control apparatus 116 is mounted on the tube assembly. The tube control apparatus 116 includes user interfaces such as a display screen and a control button for performing preparation work before image capture, such as patient selection, protocol selection, positioning, etc.

The movement of the suspension apparatus 110 includes the movement of the tube assembly along the x-axis, y-axis, and z-axis, as well as the rotation of the tube assembly in a horizontal plane (the axis of rotation is parallel to or coincides with the z-axis) and in a vertical plane (the axis of rotation is parallel to the y-axis). In the described movement, a motor is usually used to drive a rotary shaft which in turn drives a corresponding component to rotate, so as to achieve a corresponding movement or rotation, and a corresponding control component is generally mounted in the sliding member 114. An X-ray imaging unit further includes a motion control unit (not shown in the figure), and the motion control unit can control the described movement of the suspension apparatus 110. Furthermore, the motion control unit can receive a control signal to control a corresponding component to move correspondingly.

The wall stand apparatus 120 includes a first detector assembly 121, a wall stand (e.g., a chest radiography stand) 122, and a connecting portion 123. The connecting portion 123 includes a support arm that is vertically connected in the height direction of the wall stand 122 and a rotating bracket that is mounted on the support arm, and the first detector assembly 121 is mounted on the rotating bracket. The wall stand apparatus 120 further includes a detector driving apparatus that is provided between the rotating bracket and the first detector assembly 121. Under the drive of the detector driving apparatus, the first detector assembly 121 moves along a direction that is parallel to the height direction of the wall stand 122 in a plane that is supported by the rotating bracket, and the first detector assembly 121 may be further rotated relative to the support arm to form an angle with the wall stand. The first detector assembly 121 has a plate-like structure the orientation of which can be changed, so that the incident surface of the X-rays becomes vertical or horizontal depending on the incident direction of the X-rays.

A bed plate assembly 132 and a second detector assembly 131 are included on the examination bed apparatus 130. The second detector assembly 131 includes a moving assembly and an accommodation portion. The moving assembly is used to drive the accommodation portion (e.g., a tray) below the bed plate assembly 132 and a detector panel to move longitudinally along a bed plate. The accommodation portion may be used to accommodate and carry the detector panel. Specifically, the receiving range of the detector panel can be along the longitudinal direction from one side of a bed panel to the other side. Specifically, the moving assembly includes a synchronous belt, a guide rail, and a motor. The guide rail is arranged in the longitudinal direction. The accommodation portion (e.g., the tray) can move relative to the guide rail. One end of the synchronous belt is fixed on the accommodation portion (e.g., the tray), and the other end is connected to the motor, so as to control the synchronous belt by means of the motor to drive the accommodation portion (e.g., the tray) to move, so that the accommodation portion (e.g., the tray) and the detector move in the longitudinal direction. The described movement configuration enables the detector panel to cover the position of the entire bed panel, so that any position on the subject under examination can be imaged, or image splicing can be performed on a plurality of positions.

Alternatively, however, the detector panel in the second detector assembly 131 may be detached from the second detector assembly 131, placed on a separate support or held by the subject under examination, for use in an exposure mode in which the position of the detector is freely adjustable. The selection or use of the first detector assembly 121 and the second detector assembly 131 may be determined on the basis of an image capture site of a patient and/or an image capture protocol, or may be determined on the basis of the position of the subject under examination that is obtained by the capturing of a camera. FIG. 1 merely shows a schematic diagram of a wall stand and an examination bed. It should be understood by those skilled in the art that a wall stand and/or examination bed in any form or arrangement may be selected or just the wall stand can be mounted, and the wall stand and/or examination bed do/does not limit the entire solution of the present application.

In some embodiments, the control apparatus 150 may include a source controller and a detector controller. The source controller is used to command the X-ray source to emit X-rays for image exposure. The detector controller is used to select a suitable detector among a plurality of detectors, and to coordinate the control of various detector functions, such as automatically selecting a corresponding detector according to the position or pose of the subject under examination. Alternatively, the detector controller may perform various signal processing and filtering functions, specifically, for initial adjustment of a dynamic range, interleaving of digital image data, and the like. In some embodiments, the control apparatus may provide power and timing signals for controlling the operation of the X-ray source and the detector.

In some embodiments, the control apparatus may also be configured to use a digitized signal to reconstruct one or more required images and/or determine useful diagnostic information corresponding to a patient, wherein the control apparatus may include one or more dedicated processors, graphics processing units (GPUs), digital signal processors, microcomputers, microcontrollers, application-specific integrated circuits (ASICs), field-programmable gate arrays (FPGAs), or other suitable processing apparatuses.

Certainly, the medical imaging system may also include other numbers, configurations or forms of control apparatuses, for example, the control apparatus may be local (e.g., co-located with one or more medical imaging systems 100, such as within the same facility and/or the same local network). In other implementations, the control apparatus may be remote, and thus only accessible by means of a remote connection (for example, by means of the Internet or other available remote access technologies). In a specific implementation, the control apparatus may also be configured in a cloud-like means, and may be accessed and/or used in a means that is substantially similar to the means by which other cloud-based systems are accessed and used.

The system 100 also includes a storage apparatus (not shown in the figure). A processor may store the digitized signal in a memory. For example, the memory may include a hard disk drive, a floppy disk drive, a CD-read/write drive, a digital versatile disc (DVD) drive, a flash drive, and/or a solid-state memory. The memory may also be integrated together with the processor to effectively use the footprint and/or meet expected imaging requirements.

The system 100 further includes an input apparatus 160. The input apparatus 160 may include a certain form of operator interface, such as a keyboard, a mouse, a voice-activated control apparatus, a touch screen (which may also be used a display apparatus described later), a tracking ball or any other suitable input device. An operator may input an operating signal/control signal to the control apparatus by means of the input device.

The system 100 further includes a display apparatus 151 (such as a touch screen or a display screen). The display apparatus 151 may be used to display an operation interface such as a list of subjects under examination, the positioning or exposure configurations of the subjects under examination, and images of the subjects under examination.

In some embodiments, the medical imaging system may further include an image capture apparatus 140 (such as an optical camera). The subject under examination may be captured by the image capture apparatus to obtain a captured image that includes the subject under examination, for example a static image or a series of image frames in a dynamic real-time video stream, to carry out auxiliary positioning and exposure configurations and the like. The image capture apparatus may be mounted on the suspension apparatus, for example mounted on a side edge of the beam limiter 117, and the like, but the embodiments of the present application are not limited thereto.

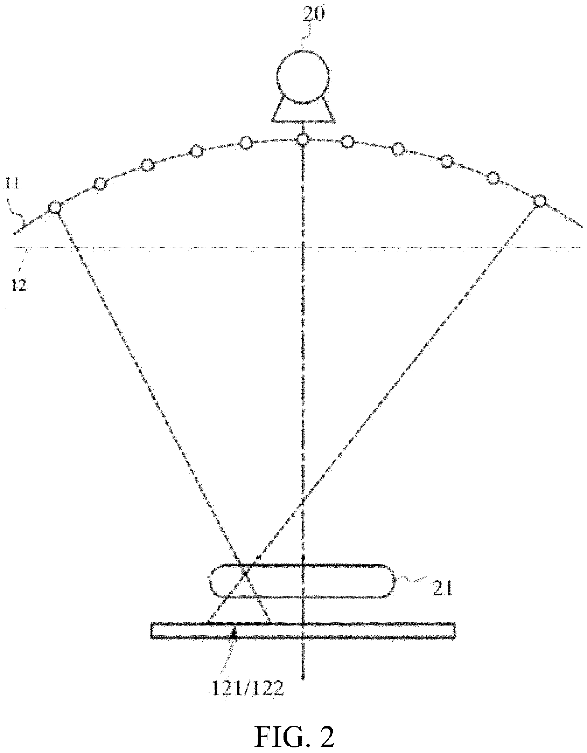

In TOMO technology, a plurality of 2D projection images of a body part are acquired from a plurality of angles during scanning, and a projection image at each angle needs to be acquired after exposure. FIG. 2 is a schematic diagram of tomosynthesis according to an embodiment of the present application. Referring to FIG. 2, during tomosynthesis scanning, a patient 21 positioned at the wall stand 122 and scanned is used as an example. An X-ray source 20 moves along the trajectory of an arc 11 that may be centered above a top surface of the first detector assembly 121, or may translate along a linear trajectory 12 in a parallel plane opposite the first detector assembly 121. At predetermined discrete positions, the X-ray source 20 is excited so as to emit a collimated X-ray beam, for example, but not limited to, at each predetermined angle on the trajectory. The first detector assembly 121 and associated electronic equipment generate digital image data for each pixel of a rectangular pixel grid at each predetermined discrete angular position of the X-ray source 20. That is, after exposure at each predetermined discrete angular position, a projection image corresponding to the angle is acquired.

Movement of the X-ray source 20 may be continuous or discontinuous. In addition, the movement of the X-ray source 20 is not necessarily along an arc or a translation, and may also be a combination of translations or different types of movement, such as partial translation and partial rotation.

For each different piece of image data, a feature of a subject under examination is projected onto the detector at a different position, and each piece of image data corresponds to a different X-ray source position. The X-ray exposure is performed in a temporally and spatially uniform manner. However, if the patient moves during scanning, then the projection of the feature also shifts.

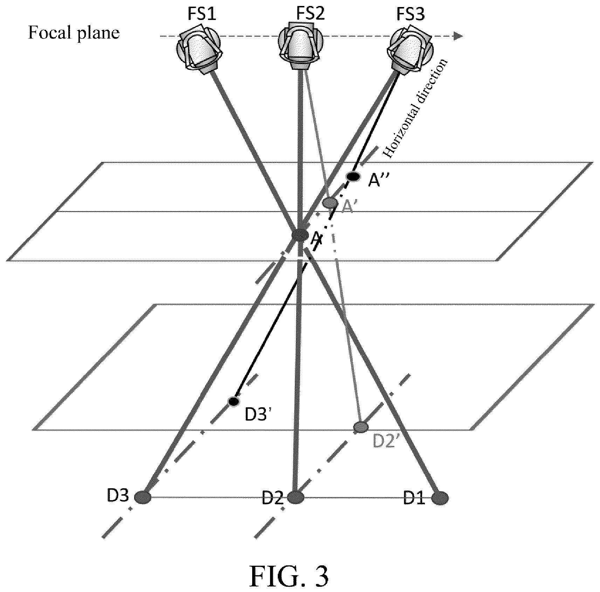

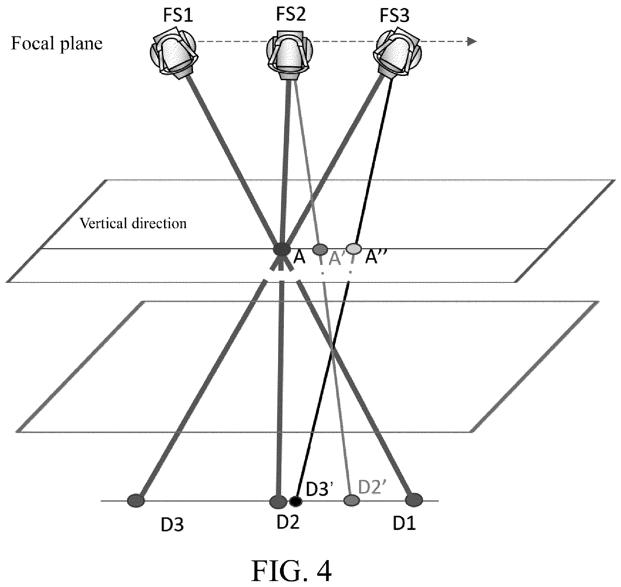

FIG. 3 and FIG. 4 are schematic diagrams of a reconstructed image affected by motion of a subject according to an embodiment of the present application. As shown in FIG. 3 and FIG. 4, on a focal plane, the X-ray source translates along a linear trajectory. As shown in FIG. 3, the subject moves in a direction perpendicular to the aforementioned trajectory. If no movement occurs, projection points D1, D2 and D3 all originate from a feature point A. However, due to movement from A to A' to A", the projection points are actually D1, D2' and D3'. As shown in FIG. 4, the subject moves in a direction parallel to the aforementioned trajectory. If no movement occurs, projection points D1, D2 and D3 all originate from a feature point A. However, due to movement from A to A' to A", the projection points are actually D1, D2' and D3'. That is, the actual position of the subject or feature under examination is different from the theoretical position of the subject or feature under examination.

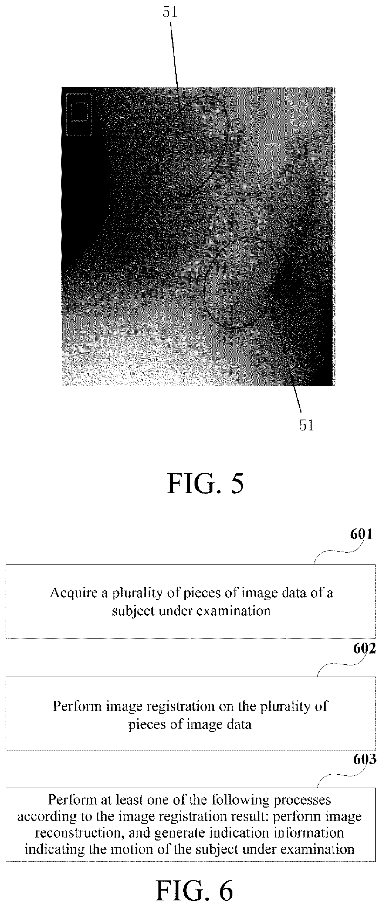

The inventors have found that the time from the start of exposure to the end of the last exposure is about a dozen seconds, and in order to ensure that the subject under examination does not move during scanning and affect image quality, the subject under examination needs to remain stationary for about a dozen seconds, otherwise movement of the subject under examination may cause artifacts in a reconstructed image. If the subject under examination is scanned again due to the occurrence of an artifact in the image, more inconvenience is incurred by the patient. FIG. 5 is a schematic diagram of an existing reconstructed image. As shown in FIG. 5, for a lateral image of the cervical spine, the patient moves significantly during acquisition, e.g., the patient moves about 15 mm horizontally from left to right, and the distance from the right corner of the edge of the C3 vertebra to the detector boundary varies from 131 mm to 116 mm from projection image #1 to projection image #30, so that significant image artifacts occur at the vertebra on the reconstructed image, and even the problem of a double edge indicated by reference numeral 51 in FIG. 5 appears.

Addressing at least one of the described problems, the embodiments of the present application provide a medical image processing method and a medical imaging system. The embodiments of the present application are described below in detail.

Provided in the embodiments of the present application is a medical image processing method. FIG. 6 is a schematic diagram of the medical image processing method of an embodiment of the present application. As shown in FIG. 6, the method includes:
601, Acquiring a plurality of pieces of image data of a subject under examination;
602, Performing image registration on the plurality of pieces of image data; and
603, Performing at least one of the following processes according to the image registration result: performing image reconstruction, and generating indication information indicating the motion of the subject under examination.

In some embodiments, the plurality of (M, M being an integer greater than 1) pieces of image data are a plurality of pieces of original projection image data of a plurality of orientations acquired by means of a detector. The original projection image data is, for example, two-dimensional image data. For example, an X-ray source moves along the trajectory of an arc or translates along a linear trajectory. At M predetermined discrete positions, the X-ray source is excited so as to emit a collimated X-ray beam, and the detector receives an X-ray and performs exposure, to acquire M corresponding pieces of original projection image data.

In some embodiments, the plurality of (M, M being an integer greater than 1) pieces of image data are a plurality of pieces of image data acquired in a plurality of orientations by an optical camera. The image data is, for example, two-dimensional RGB image data. For example, a medical imaging system includes a plurality of (M) optical cameras located at different positions, and the plurality of optical cameras each capture a subject under examination to acquire a corresponding plurality of pieces of RGB image data. Alternatively, the medical imaging system includes a positionally movable optical camera (for example, mounted on a suspension apparatus), and the optical camera is controlled to sequentially move to the plurality of orientations to capture images, to acquire a corresponding plurality of pieces of two-dimensional RGB image data, respectively. The manner of movement of the optical camera is similar to that of the aforementioned X-ray source, and will not be repeated here.

In some embodiments, the image data may be represented as a two-dimensional array. For ease of description, the plurality of pieces of image data are represented as I1, I2, I3, ..., IM. I1, I2, I3, ..., IM may be arranged according to a temporal order of acquisition of the image data, but the embodiments of the present application are not limited thereto.

In some embodiments, unlike in the prior art where image registration is carried out using reconstructed images, in 602, image registration is performed on the plurality of pieces of original projection image data or the plurality of pieces of RGB image data. That is, image registration is performed using the plurality of pieces of image data, or an image registration algorithm is applied on or between the plurality of pieces of image data, thereby reducing artifacts on a reconstructed slice image caused by motion of an object.

In the embodiments of the present application, image registration may also be referred to as image alignment, which refers to determining a spatial transformation means for one piece of image data so that a corresponding point thereon spatially coincides with a corresponding point on another piece of image data. Coinciding means that an anatomical feature point on the subject under examination has the same spatial position on two pieces of image data. The result of the registration should be that all anatomical feature points on the image data, or at least all diagnostically significant anatomical feature points (positions of interest), are matched. The anatomical feature points include, but are not limited to, a tissue, an organ, a lesion, a calcification, a tumor, etc. For different image data, a mapping relationship P is sought such that for each point on an image (e.g., a first image), a unique point corresponding thereto can be found on an image (e.g., a second image), and the two points should correspond to the same anatomical position. The mapping relationship P is represented as a set of contiguous spatial transformations. Commonly used spatial geometrical transformations are rigid transformations, non-rigid transformations, or affine transformations. The rigid transformations may include, for example, a translation, a rotation, or a combination of a translation and a rotation. In addition, non-rigid transformations may include, for example, finite element modeling, a B-spline transformation, a diffusion-based method, an optical-flow based method, or a level-set based method. An affine transformation may be a uniform scale transformation in which scale transformation coefficients of all directions are consistent, a non-uniform scale transformation in which transformation coefficients are not consistent, a shear transformation, and the like. For details, reference may be made to related technologies, and details are not repeatedly described herein. It should be noted that image registration to which the embodiments of the present application are related can use any existing image registration algorithm, and the embodiments of the present application are not limited thereto.

In some embodiments, image registration may be performed using global data of each piece of image data. A result of the image registration includes obtaining a shift vector characterizing the motion of the subject under examination. The shift vector includes parameters characterizing the motion of the subject under examination, such as a horizontal shift, a vertical shift, rotation angle, etc. The image registration may be rigid registration, which is merely an example, and the embodiments of the present application are not limited thereto. The image registration may also be non-rigid registration, or the like, and examples are not listed herein one by one again.

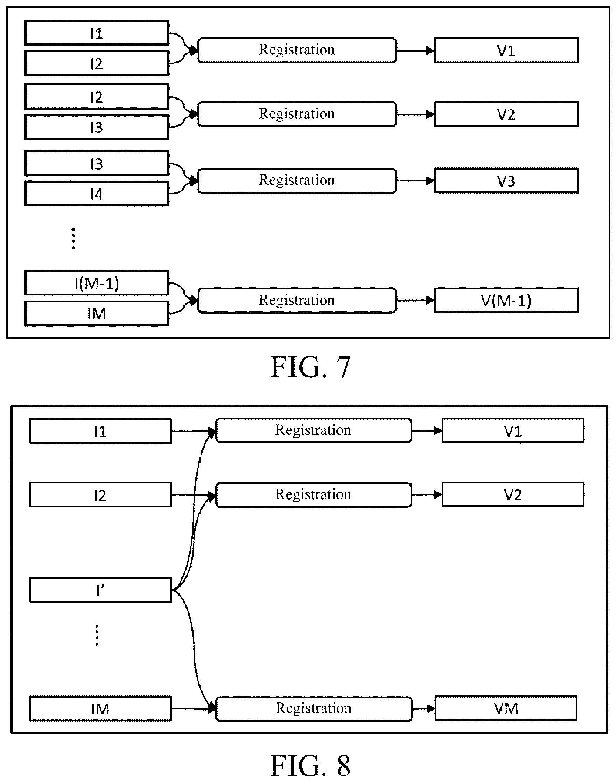

As an implementation means (I), image registration may be performed on every two adjacent pieces of image data among the M pieces of image data. FIG. 7 is a schematic diagram of image registration according to embodiments of the present application. As shown in FIG. 7, global image registration is separately performed on every two adjacent pieces of image data among I1 to IM, for example, I1 and I2, I2 and I3, ..., and I(M - 1) and IM, and a registration result of every two adjacent pieces of image data includes obtaining one shift vector. Therefore, the registration results of I1 and I2, I2 and I3, ..., and I(M - 1) and IM include obtaining M-1 shift vectors V1, V2, ..., and V(M - 1). As the motion of the subject under examination in adjacent image data is relatively insignificant, the rigid registration method is a more precise and more convenient registration method. However, the embodiments of the present application are not limited thereto.

As another implementation means (II), image registration may be performed on each piece of image data among the M pieces of image data and reference image data. The reference image data is one of the plurality of pieces of image data, and said one piece of image data may be the first piece of image data (I1) among the M pieces of image data or image data located at a middle position among the M pieces of image data (for example, when the X-ray source is perpendicular to the detector), or the reference image data is image data used for scouting, to which the embodiments of the present application are not limited. The image data used for scouting is used in a conventional scanning procedure for low-resolution imaging performed on the subject under examination before high-resolution imaging is performed on a region of interest, and one objective thereof is to more precisely determine the region of interest in an acquired positioning image. FIG. 8 is a schematic diagram of image registration according to an embodiment of the present application. As shown in FIG. 8, image registration is separately performed on each piece of image data among I1 to IM, as well as reference image data 1'. For example, global image registration is separately performed on I1 and I', I2 and I', ..., and IM and I'. A result of the registration performed on each pair of pieces of image data includes obtaining one shift vector, and therefore registration results of I1 and I', I2 and I', ..., and IM and I' include obtaining M shift vectors V1, V2, ..., and VM. The position of the subject under examination in the reference image data may be used as a reference position, and other image data can be registered with the reference image data, thereby further improving registration precision.

In the above two implementation means, the image data may be two-dimensional RGB image data or two-dimensional original projection image data, to which the embodiments of the present application are not limited.

In some embodiments, image registration may be performed using local data in each piece of image data. The result of the image registration includes obtaining a shift vector representing the motion of the subject under examination. The image registration may be rigid registration, which is merely an example, and the embodiments of the present application are not limited thereto. The image registration may also be non-rigid registration, or the like, and examples are not listed herein one by one again.

In some embodiments, the local data may be data of a preset position. For example, the preset position may be a middle position of the image data or a predetermined anatomical position, the anatomical position including a tissue, an organ, a lesion, a calcification, a tumor, etc.

As an implementation means (III), image registration may be performed on data at preset positions of every two adjacent pieces of image data among the M pieces of image data. For example, local image registration is separately performed on data at preset positions of every two adjacent pieces of image data among I1 to IM, for example, I1 and I2, I2 and I3, ..., and I(M-1) and IM, and a registration result of the data at the preset positions of every two adjacent pieces of image data includes obtaining one shift vector. Therefore, the registration results of the data at the preset positions of I1 and I2, I2 and I3, ..., and I(M-1) and IM include obtaining M-1 shift vectors V1, V2, ..., and V(M-1). As the motion of the subject under examination in adjacent image data is relatively insignificant, the rigid registration method is a more precise and more convenient registration method. However, the embodiments of the present application are not limited thereto.

As another implementation means (IV), image registration may be performed on data at a preset position in each piece of image data among the M pieces of image data and data at a preset position in the reference image data. The reference image data is one of the plurality of pieces of image data, and said one piece of image data may be the first piece of image data (I1) among the M pieces of image data, or image data located at a middle position among the M pieces of image data (for example, when the X-ray source is perpendicular to the detector), or the reference image data is image data used for scouting. The embodiments of the present application are not limited thereto. For example, image registration is separately performed on data at a preset position in each piece of image data among I1 to IM and data at a preset position in reference image data I'. For example, local image registration is respectively performed on I1 and I', I2 and I', ..., and IM and I'. A result of the registration performed on the data at the preset position in each pair of image data includes obtaining one shift vector, and thus registration results of the data at the preset position in I1 and I', I2 and I', ..., and IM and I' include obtaining M shift vectors V1, V2, ..., and VM. The position of the subject under examination in the reference image data may be used as a reference position, and other image data can be registered with the reference image data, thereby further improving registration precision.

In the above two implementation means, the image data may be two-dimensional optical image data or two-dimensional original projection image data, to which the embodiments of the present application are not limited.

In some embodiments, the local data may be determined on the basis of a reconstructed image (a 3D slice image); in said embodiments, the image data is the original projection image data. A predetermined anatomical position (hereinafter referred to as a position of interest) in the reconstructed image may be determined. The anatomical position includes a tissue, an organ, a lesion, a calcification, a tumor, etc., and it is determined using, for example, a forward projection method that said anatomical position is reconstructed from data of which projection positions in which original projection image data among the M pieces of original projection image data. In other words, it is determined which projection positions in which original projection image data among the M pieces of original projection image data contribute to obtaining the position of interest in the 3D slice image, and image registration is performed on the basis of the determined projection positions.

The detailed description is provided below.

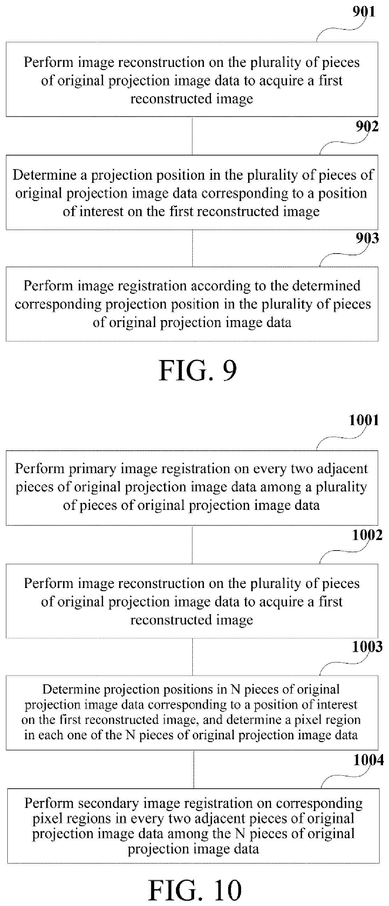

In some embodiments, the plurality of pieces of image data are a plurality of pieces of original projection image data of a plurality of orientations acquired by means of a detector, and are also referred to as a plurality of pieces of tomosynthesis projection data. FIG. 9 is a schematic diagram of step 602 according to an embodiment of the present application. As shown in FIG. 9, step 602 includes:
901, Performing image reconstruction on the plurality of pieces of original projection image data to acquire a first reconstructed image;
902, Determining a projection position in the plurality of pieces of original projection image data and corresponding to a position of interest on the first reconstructed image; and
903, Performing image registration according to the determined corresponding projection position in the plurality of pieces of original projection image data.

In some embodiments, in 901, image reconstruction (tomosynthesis) is performed on the plurality of pieces of original projection image data to acquire the first reconstructed image, and the image reconstruction algorithm may include: a back projection (FBP) reconstruction method, an adaptive statistical iterative reconstruction (ASIR) method, a conjugate gradient (CG) method, a maximum likelihood expectation maximization (MLEM) method, a model-based iterative reconstruction (MBIR) method, etc. Please refer to related technology for details, and the embodiments of the present application are not limited thereto.

In some embodiments, in 902, a position of interest, e.g., a tissue, an organ, a lesion, a calcification, a tumor, etc., is determined on the first reconstructed image. For example, positions of interest determined on the first reconstructed image are Y1, Y2, ..., and YK (a pixel in one 3D slice image is used as one position, and the positions of interest include a plurality of pixel positions), and projection positions in the plurality of pieces of original projection image data and corresponding to the positions of interest are determined using the forward projection method. The original projection image data contributing to acquisition of the positions of interest may be the N pieces of original projection image data, where N is less than or equal to M.

In some embodiments, in 903, a pixel region in each one of the N pieces of original projection image data is determined, the pixel region including the projection position determined in 902. For example, the projection position may be preset to be a vertex position of an edge of the pixel region or a center position of the pixel region, to which the embodiments of the present application are not limited. The size of the pixel region may be determined on the basis of a scanning protocol performed on the subject under examination. For example, when the subject under examination is a radiograph of the chest, the pixel region may be configured to be larger, and when the subject under examination is a vertebral body, the pixel region may be configured to be smaller, to which the embodiments of the present application are not limited. The positions of pixel regions corresponding to the projection positions in the same piece of original projection image data determined on the basis of the different positions of interest are different (the sizes may be the same or different).

In some embodiments, in 903, image registration is performed according to corresponding pixel regions in the N pieces of original projection image data. The pixel regions corresponding to the N pieces of original projection image data may be represented as W1, W2, ..., WN.

As an implementation means (V), image registration is performed on corresponding pixel regions in every two adjacent pieces of original projection image data among the N pieces of original projection image data. For example, image registration is performed on W1 and W2, W2 and W3, ..., and W(N-1) and WN, respectively.

As another implementation means (VI), image registration is performed on a corresponding pixel region in each one of the N pieces of original projection image data and a corresponding pixel region in reference image data. The reference image data is one piece of the pieces of original projection image data, and the one piece of original projection image data may be the first piece of original projection image data among the M pieces of original projection image data, or original projection image data located at a middle position, or original projection image data used for scouting, to which the embodiments of the present application are not limited. For example, image registration is separately performed on each pixel region in W1 to WN and a pixel region W' in the reference image data. For example, image registration is separately performed on W1 and W', W2 and W', ..., and WM and W'.

For image registration methods in the above two implementation means, reference may be made to related technology, and the embodiments of the present application are not limited thereto. As an example, for two pixel regions, anatomical feature points in the different pixel regions are respectively identified. For extraction of the feature points, reference may be made to related technology, and the embodiments of the present application are not limited thereto. For example, identification is performed by using a neural network model or the like. A similarity measure (a similarity matrix) between the anatomical feature points in the different pixel regions is calculated to determine matching feature points in different image data. The similarity measure may also be determined on the basis of methods such as cross-correlation and entropy. The similarity measure is maximized, and the anatomical feature points corresponding to the largest similarity measure are used as matching feature points. A result of the image registration is the positions of feature points that actually match in each pixel region. This is merely an example, and other image registration methods are also applicable to the present application, and examples are not listed herein one by one again.

The positions of interest Y1, Y2, ..., and YK are used as an example. For Y1, it is determined by means of forward projection that projection positions on the N pieces of original projection image data corresponding to Y1 are P1, P2, P3, ..., and PN, respectively and pixel regions W1, W2, ..., and WN on the N pieces of original projection image data and corresponding to P1, P2, P3, ..., and PN, respectively are determined. Image registration is separately performed on W1 and W2, W2 and W3, ..., and WN-1 and WN, or is respectively performed on W1 and W', W2 and W', ..., and WM and W'. For example, for the two pixel regions W1 and W2, in 902, the anatomical feature point P1 in the first pixel region W1 is determined, and the largest similarity measure is calculated, thereby acquiring the feature point P2' in the second pixel region W2 which matches P1. Due to motion of the subject under examination, the matching feature point P2' may be different from the projection position P2 in the second pixel region W2 determined in 902. For each two pixel regions, the feature points that actually match are determined using the method described above. For the other positions of interest Y2, ..., YK, matching feature points respectively corresponding thereto are determined by the same implementation means as Y1, which will not be repeated here.

How to perform image registration using image data in the embodiments of the present application is described above. The implementation means (I) to (VI) for image registration may be implemented independently, or at least two implementation means may be implemented in combination, and the embodiments of the present application are not limited thereto. Below, two implementation means are combined as an example for description.

FIG. 10 is a schematic diagram of an image registration method according to an embodiment of the present application. As shown in FIG 10, the method includes:
1001, Performing primary image registration on every two adjacent pieces of original projection image data among a plurality of pieces of original projection image data;
1002, Performing image reconstruction on the plurality of pieces of original projection image data to acquire a first reconstructed image;
1003, Determining projection positions in N pieces of original projection image data corresponding to a position of interest on the first reconstructed image, and determining a pixel region in each one of the N pieces of original projection image data; and
1004, Performing secondary image registration on corresponding pixel regions in every two adjacent pieces of original projection image data among the N pieces of original projection image data.

An example of implementation means of 1001 to 1004 is performing registration on adjacent image data. The image registration in 1001 to 1004 may also be registration performed on each piece of image data and reference image data. The specific embodiments are as described above and will not be repeated here. In addition, the primary image registration and the secondary image registration are not performed in a particular order, and the embodiments of the present application are not limited thereto.

In some embodiments, the particular implementation means used for image registration may depend on the scanning protocol. For example, for scanning of a vertebral body, the implementation means (I) may be used by default for image registration. Examples are not listed herein one by one again.

The following describes how to perform subsequent processing according to a result of image registration.

In some embodiments, at least one of the following processes may be executed according to the result of the image registration: performing image reconstruction, and generating indication information indicating the motion of the subject under examination.

As an implementation means, the indication information is generated according to the result of the image registration, and the indication information may be represented as a vector mark indicating the magnitude and direction of motion of the subject under examination, a trajectory map indicating motion of the subject under examination, a coordinate system indicating the magnitude and direction of motion of the subject under examination, or the like, to which the embodiments of the present application are not limited.

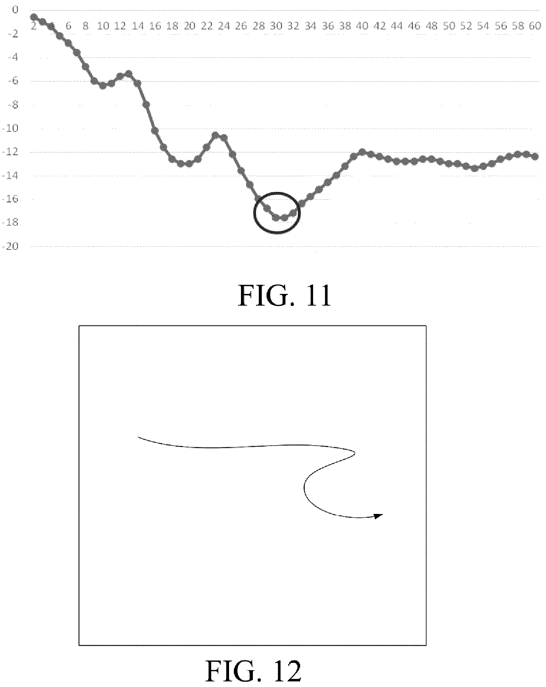

For example, the indication information may be generated by determining the magnitude and direction of motion of the subject under examination on the basis of the shift vector, or the indication information may be generated on the basis of the determined position of the feature point actually matching in each pixel region, to which the embodiments of the present application are not limited. FIG. 11 and FIG. 12 are schematic diagrams of indication information according to embodiments of the present application. As shown in FIG. 11, the abscissa represents M pieces of image data acquired according to a temporal order, and the ordinate represents a relative position (for example, the horizontal direction) relative to a start position of the subject under examination in the first piece of original projection image data determined according to the shift vector. A positive number indicates that the subject moves from right to left, and a negative number indicates that the subject moves from left to right. At the 30th piece of image data, the subject moves 18 mm horizontally from left to right. As shown in FIG. 12, the determined positions of the feature points that actually match in the pixel regions are connected by means of a fitting curve to generate a trajectory map of the motion of the subject under examination, and the arrow indicates the direction of motion. Optionally, the indication information may be superimposed and displayed on the reconstructed image. According to the indication information, an operator can visually determine the motion of the subject under examination during scanning.

As an implementation means, image reconstruction is performed according to the result of the image registration. For example, with respect to the aforementioned implementation means (I), (II), (III) and (IV), primary image reconstruction may be performed on the basis of the aforementioned shift vector in combination with the M pieces of original projection image data, to generate a corrected reconstructed 3D slice image. Alternatively, with respect to the aforementioned implementation means (V) and (VI), an interpolation algorithm may be used on the basis of grayscale values of the determined positions of the feature points that actually match in the respective pixel regions to acquire pixel points on the reconstructed image corresponding thereto, and secondary image reconstruction is performed to generate a corrected reconstructed 3D slice image. That is, before an image is reconstructed, the motion of the subject is pre-compensated or corrected according to the result of the image registration, so as to reconstruct a 3D slice image that is not affected by the motion of the subject; this is for illustrative purposes only, and the embodiments of the present application are not limited thereto. The image reconstruction performed according to the result of the image registration has a more precise motion compensation or correction effect than conventional image reconstruction, thereby improving image quality. The image reconstruction algorithm may include: a back projection (FBP) reconstruction method, an adaptive statistical iterative reconstruction (ASIR) method, a conjugate gradient (CG) method, a maximum likelihood expectation maximization (MLEM) method, a model-based iterative reconstruction (MBIR) method, etc. Please refer to related technology for details, and the embodiments of the present application are not limited thereto.

In some embodiments, just the indication information can be generated according to the result of the image registration, and optionally, the indication information may be further displayed in a graphical user interface of a medical imaging system (in this case, image reconstruction is performed on the basis of a plurality of pieces of original projection image data). Alternatively, just the image reconstruction can be performed according to the result of the image registration (no indication information is generated). Alternatively, according to the result of the image registration, not only the indication information is generated, but the image reconstruction is also performed. Alternatively, the indication information may be generated according to the result of the image registration, and the operator may determine the motion of the subject under examination according to the indication information. Further, it is automatically or manually determined whether the image reconstruction needs to be performed with reference to the result of the image registration, or that the image reconstruction is performed only on the basis of the plurality of pieces of original projection image data.

In the case of manual determination, the graphical user interface of the medical imaging system further includes a reconstruction icon. When the reconstruction icon is operated (clicked by the operator), image reconstruction is performed according to the result of the image registration in combination with the plurality of pieces of original projection image data. In the case of automatic determination, the displacement of the motion of the subject may be determined according to the result of the image registration, and the displacement may be compared with a preset threshold. If the displacement exceeds the threshold, the image reconstruction is performed according to the result of the image registration in combination with the plurality of pieces of original projection image data; otherwise, the image reconstruction is performed only on the basis of the plurality of pieces of original projection image data.

In some embodiments, whether the image reconstruction needs to be performed by default according to the result of the image registration may depend on the scanning protocol. For example, when an infant is scanned or when a patient who is standing is scanned, the probability that movement will occur is high, and thus during the relevant scan, image reconstruction may be performed by default according to a result of image registration in combination with a plurality of pieces of original projection image data. Examples are no longer listed herein one by one.

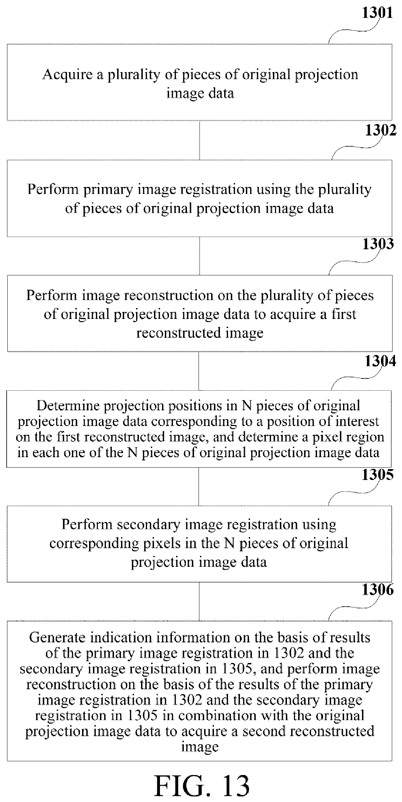

FIG. 13 is a schematic diagram of a medical image processing method according to embodiments of the present application. Image data is, for example, original projection image data. As shown in FIG. 13, the method includes:
1301, Acquiring a plurality of pieces of original projection image data;
1302, Performing primary image registration using the plurality of pieces of original projection image data;
1303, Performing image reconstruction on the plurality of pieces of original projection image data to acquire a first reconstructed image;
1304, Determining projection positions in N pieces of original projection image data and corresponding to a position of interest on the first reconstructed image, and determining a pixel region in each one of the N pieces of original projection image data;
1305, Performing secondary image registration using the corresponding pixel regions in the N pieces of original projection image data; and
1306, Generating indication information on the basis of results of the primary image registration in 1302 and the secondary image registration in 1305, and performing image reconstruction on the basis of the results of the primary image registration in 1302 and the secondary image registration in 1305 in combination with the original projection image data to acquire a second reconstructed image.

Implementation means of 1301 to 1306 are as described above, and will not be described again here. For the primary registration in 1302, reference may be made to any one of the foregoing implementation means (I) to (IV). For the secondary registration in 1305, reference may be made to the foregoing implementation means (V) or (VI). The order of performing the primary registration and the secondary registration is not limited. In addition, just the primary registration (1302) may be performed, or just the secondary registration (1303 to 1305) can be performed, to which the embodiments of the present application are not limited.

It should be noted that the foregoing accompanying drawings merely schematically illustrate embodiments of the present application, but the present application is not limited thereto. For example, the order of execution of operations may be appropriately adjusted. In addition, some other operations may be added or some operations may be omitted. Alternatively, operations may be added. Those skilled in the art can make appropriate variations according to the above content, rather than being limited by the disclosure of the foregoing accompanying drawings.

The above embodiments merely provide illustrative descriptions of the embodiments of the present application. However, the present application is not limited thereto, and appropriate variations may be made on the basis of the above embodiments. For example, each of the above embodiments may be used independently, or one or more among the above embodiments may be combined.

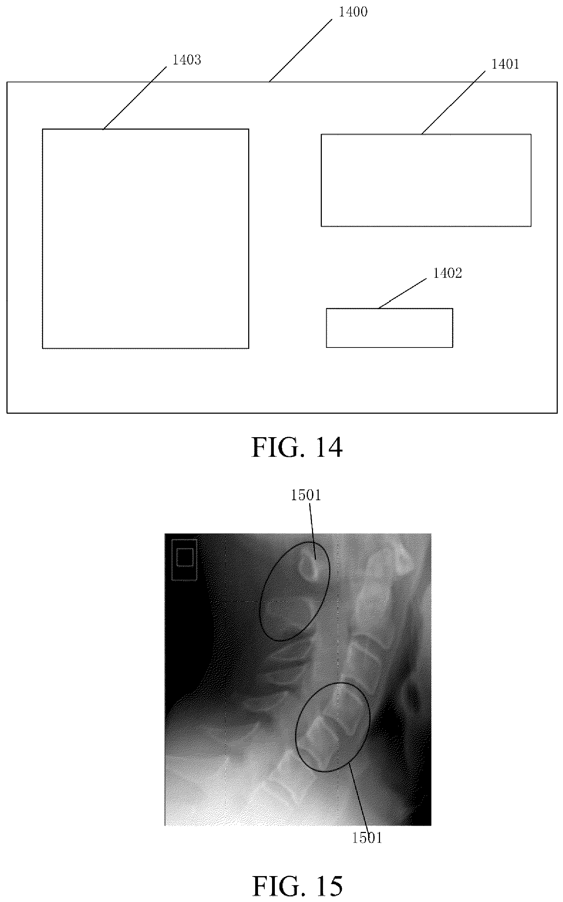

FIG. 15 is a schematic diagram of a reconstructed image according to an embodiment of the present application. As shown in FIG. 15, a marker 1501 is compared with the marker 51 in FIG. 5, and according to the embodiments of the present application, image registration is performed by using acquired original image data, thereby reducing artifacts on a reconstructed slice image caused by movement of an object, improving image quality, and avoiding repeated scanning.

In addition, image registration is performed on every two adjacent pieces of image data. As the motion of the subject under examination in adjacent image data is relatively insignificant, the rigid registration method is a more precise and more convenient registration method.

In addition, image registration is performed on each piece of image data and reference image data, and the reference image data is one of the plurality of pieces of image data or image data for positioning. As a result, registration precision can be further improved.

In addition, it is determined, according to a position of interest in a reconstructed 3D slice image, which projection positions in which original projection image data among the M pieces of original projection image data correspond thereto, and image registration is performed on the basis of the determined projection positions. As a result, registration precision can be further improved.

Embodiments of the present application further provide a medical imaging system. The medical imaging system includes: an acquisition device, configured to acquire a plurality of pieces of image data of a subject under examination; and a controller, configured to execute the medical image processing method in the foregoing embodiments.

In some embodiments, the acquisition device includes: at least one of an imaging apparatus and an optical camera, the imaging apparatus including an X-ray source and a detector, the X-ray source and the detector being capable of cooperating to acquire a plurality of pieces of original projection image data of the subject under examination in a plurality of orientations as the plurality of pieces of image data, and the optical camera capturing the subject under examination in the plurality of orientations to acquire the plurality of pieces of image data.

In some embodiments, the medical imaging system further includes: a display apparatus, configured to display a graphical user interface. When indication information is generated according to a result of the image registration, FIG. 14 is a schematic diagram of a graphical user interface according to an embodiment of the present application. As shown in FIG. 14, the graphical user interface 1400 further displays the indication information 1401.

With respect to implementations of the imaging apparatus, the optical camera and the display apparatus, reference may be made to FIG. 1 and the related description thereof, which will not be repeated here.

In some embodiments, the graphical user interface 1400 further includes a reconstruction button 1402. When the reconstruction button is triggered, the controller performs image reconstruction according to the result of the image registration. In addition, a reconstructed image 1403 may be displayed on the graphical user interface.

The medical imaging system may further include other assemblies; for details, reference may be made to FIG. 1. Examples are not listed herein one by one again.

The medical imaging system includes, but is not limited to: a computed tomography (CT) system, a magnetic resonance imaging (MRI) system, a C-arm imaging system, a positron emission computed tomography (PET) system, a single photon emission computed tomography (SPECT) system, an ultrasonic system, an X-ray imaging system, or any other suitable medical imaging system.

Although some embodiments of the present application are described on the basis of the suspended X-ray imaging system shown in FIG. 1, the embodiments of the present application are not limited thereto. For example, the medical imaging system may also be a floor-standing X-ray imaging system, a mobile X-ray imaging system, or the like. Examples are not listed herein one by one again.

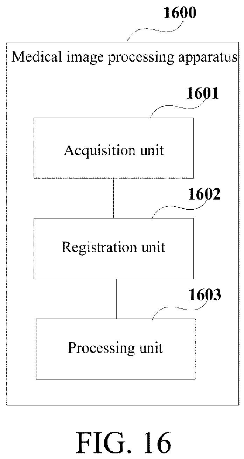

The embodiments of the present application further provide a medical image processing apparatus. FIG. 16 is a schematic diagram of a medical image processing apparatus according to an embodiment of the present application. As shown in FIG. 16, the apparatus 1600 includes:

An acquisition unit 1601, configured to acquire a plurality of pieces of image data of a subject under examination;

A registration unit 1602, configured to perform image registration on the plurality of pieces of image data; and

A processing unit 1603, configured to perform at least one of the following processes according to a result of the image registration: performing image reconstruction, and generating indication information indicating the motion of the subject under examination.

In some embodiments, for implementations of the acquisition unit 1601, the registration unit 1602, and the processing unit 1603, reference may be made to 601 to 603 of the aforementioned embodiments, which will not be described herein again.

For simplicity, the above figures only exemplarily illustrate the connectional relationship or signal direction between various components or modules, but it should be clear to those skilled in the art that various related technologies such as bus connection can be used. The various components or modules can be implemented by means of hardware such as a processor or a memory, etc. The embodiments of the present application are not limited thereto.

The above embodiments merely provide illustrative descriptions of the embodiments of the present application. However, the present application is not limited thereto, and appropriate variations may be made on the basis of the above embodiments. For example, each of the above embodiments may be used independently, or one or more among the above embodiments may be combined.

The embodiments of the present application further provide a medical image processing device. The medical image processing device may include: one or more processors (for example, a central processing unit (CPU)) and one or more memories. The memory is coupled to the processor. The memory can store a control program, and is controlled by the processor to execute the program. The memory may include, for example, a ROM, a floppy disk, a hard disk, an optical disk, a magneto-optical disk, a CD-ROM, or a non-volatile memory card.

In some embodiments, functions of the medical image processing apparatus are integrated into and implemented by the processor. The processor is configured to implement the medical image processing method as described in the aforementioned embodiments. For the implementation of the processor, reference may be made to the aforementioned embodiments, which will not be described again here.

In some embodiments, the medical image processing apparatus and the processor are configured separately, for example, the medical image processing apparatus can be configured as a chip connected to the processor and the functions of the medical image processing apparatus can be achieved by means of the control of the processor.

In addition, the medical image processing device may further include: an input device, a display (displaying a graphical user interface, and various data generated during data acquisition and processing), etc. The functions of said components are similar to those in the prior art, and are not described again here.

The processor may communicate with a medical device, the display or the like in response to an operation of the input device, and may also control input actions and/or the state of the input device. The processor may also be referred to as a microcontroller unit (MCU), microprocessor or microcontroller or another processor apparatus and/or logic apparatus. The processor may include a reset circuit, a clock circuit, a chip, a microcontroller, and so on. The functions of the processor may be integrated on a main board of a medical device (e.g., the processor is configured as a chip connected to a main board processor (CPU)), or may be configured independently of the main board, and embodiments of the present application are not limited thereto.

The embodiments of the present application further provide a computer-readable program, which, when executed in an apparatus or a medical imaging system, causes a computer to execute, in the medical imaging system, the medical image processing method according to the foregoing embodiments.

The embodiments of the present application further provide a computer program product, at least including a computer-readable program, wherein the computer-readable program causes a computer to execute, in a medical imaging system, the medical image processing method according to the foregoing embodiments.

The embodiments of the present application further provide a computer-readable storage medium, the computer-readable storage medium including instructions, and the instructions, when executed, causing a processor to be configured to execute the medical image processing method according to the foregoing embodiments.

The above apparatus and method of the present application can be implemented by hardware, or can be implemented by hardware in combination with software. The present application relates to the foregoing type of computer-readable program. When executed by a logic component, the program causes the logic component to implement the foregoing apparatus or a constituent component, or causes the logic component to implement various methods or steps as described above. The present application further relates to a storage medium for storing the above program, such as a hard disk, a magnetic disk, an optical disk, a DVD, a flash memory, etc.

The method/apparatus described with reference to the examples of the present application may be directly embodied as hardware, a software module executed by a processor, or a combination of the two. For example, one or more of the functional block diagrams and/or one or more combinations of the functional block diagrams as shown in the drawings may correspond to either software modules or hardware modules of a computer program flow. The foregoing software modules may respectively correspond to the steps shown in the figures. The foregoing hardware modules may be implemented, for example, by firming the foregoing software modules by using a field-programmable gate array (FPGA).

The software modules may be located in a RAM memory, a flash memory, a ROM memory, an EPROM memory, an EEPROM memory, a register, a hard disk, a removable disk, a CD-ROM, or any storage medium in other forms known in the art. The storage medium may be coupled to a processor, so that the processor can read information from the storage medium and can write information into the storage medium. Alternatively, the storage medium may be a constituent component of the processor. The processor and the storage medium may be located in an ASIC. The software module may be stored in a memory of a mobile terminal, and may also be stored in a memory card that can be inserted into a mobile terminal. For example, if a device (such as a mobile terminal) uses a large-capacity MEGA-SIM card or a large-capacity flash memory apparatus, then the software modules may be stored in the MEGA-SIM card or the large-capacity flash memory apparatus.

One or more of the functional blocks and/or one or more combinations of the functional blocks shown in the drawings may be implemented as a general-purpose processor, a digital signal processor (DSP), an application-specific integrated circuit (ASIC), a field-programmable gate array (FPGA) or other programmable logic devices, a discrete gate or transistor logic device, a discrete hardware assembly, or any appropriate combination thereof, which is used for implementing the functions described in the present application. The one or more functional blocks and/or the one or more combinations of the functional blocks shown in the drawings may also be implemented as a combination of computing equipment, such as a combination of a DSP and a microprocessor, a plurality of microprocessors, one or more microprocessors in communication combination with a DSP, or any other such configuration.

The present application is described above with reference to specific embodiments. However, it should be clear to those skilled in the art that the foregoing description is merely illustrative and is not intended to limit the scope of protection of the present application. Various variations and modifications may be made by those skilled in the art according to the principle of the present application, and said variations and modifications also fall within the scope of the present application.

## Claims

1. A medical image processing method, **characterized in that** the method comprises:
acquiring a plurality of pieces of image data of a subject under examination;
performing image registration on the plurality of pieces of image data; and
performing at least one of the following processes according to the image registration result: performing image reconstruction, and generating indication information indicating the motion of the subject under examination.

2. The method according to claim 1, **characterized in that** the plurality of pieces of image data are a plurality of pieces of image data acquired by an optical camera in a plurality of orientations, or are a plurality of pieces of original projection image data of a plurality of orientations acquired by a detector.

3. The method according to claim 1, **characterized in that** performing image registration on the plurality of pieces of image data comprises:
performing image registration on every two adjacent pieces of image data.

4. The method according to claim 1, **characterized in that** performing image registration on the plurality of pieces of image data comprises:
performing image registration on each piece of image data and reference image data.

5. The method according to claim 1 or 3 or 4, **characterized in that** the plurality of pieces of image data are a plurality of pieces of original projection image data of a plurality of orientations acquired by a detector, and performing image registration on the plurality of pieces of image data comprises:
performing image reconstruction on the plurality of pieces of original projection image data to obtain a first reconstructed image;
determining a projection position in the plurality of pieces of original projection image data corresponding to a position of interest on the first reconstructed image; and
performing image registration according to the determined corresponding projection position in the plurality of pieces of original projection image data.

6. The method according to claim 5, **characterized in that** performing image registration according to the determined corresponding projection position in the plurality of pieces of original projection image data comprises:
determining a pixel region containing the projection position; and
performing image registration according to a corresponding search window in the plurality of pieces of original projection image data.

7. The method according to claim 6, **characterized in that** performing image registration according to a corresponding pixel region in the plurality of pieces of original projection image data comprises:
performing image registration on corresponding pixel regions in every two adjacent pieces of original projection image data.

8. The method according to claim 6, **characterized in that** performing image registration according to a corresponding pixel region in the plurality of pieces of original projection image data comprises:
performing image registration on a corresponding pixel region in original projection image data and a corresponding pixel region in reference image data.

9. The method according to claim 4 or 8, **characterized in that** the reference image data is one of the plurality of pieces of image data or image data for positioning.

10. The method according to claim 6, **characterized in that** the size of the pixel region is determined on the basis of a scanning protocol performed on the subject under examination.

11. The method according to claim 1, **characterized in that** the method further comprises: displaying the indication information.

12. The method according to claim 3 or 7, **characterized in that** performing image registration comprises performing rigid registration.

13. The method according to claim 3 or 4, **characterized in that** an image registration result comprises a shift vector representing the motion of the subject under examination.

14. The method according to claim 7, **characterized in that** performing image registration comprises:
identifying anatomical feature points in different pixel regions; and
calculating a similarity measure between the anatomical feature points in the different pixel regions, so as to determine matching feature points in different pieces of image data.

15. The method according to claim 14, **characterized in that** the anatomical feature points corresponding to the largest similarity measure are used as matching feature points.
